# EUROPÄISCHE PATENTANMELDUNG

(11) **EP 2 482 263 A1**
(43) Veröffentlichungstag der Anmeldung: **01.08.2012**
(21) Anmeldenummer: 12152746.9
(22) Anmeldetag: 26.01.2012
(51) Int. Cl.: G08B 21/22, G01L 1/16

(54) **Vorrichtung zur Erfassung der Belegung von Betten**

(30) Priorität: 27.01.2011 DE 202011002042 U
(71) Anmelder: Kleine, Klaus, 58802 Balve (DE); Thometzek, Andreas, 58540 Meinerzhagen (DE)
(72) Erfinder: Kleine, Klaus, 58802 Balve (DE); Thometzek, Andreas, 58540 Meinerzhagen (DE)
(74) Vertreter: Dörner, Kötter & Kollegen

(57) **Zusammenfassung**

Die Erfindung betrifft eine Vorrichtung zur Erfassung der Belegung von Betten, die eine Matratze (1) aufweisen. Sie umfasst einen Sensor (2), der mit einem Sender verbunden ist, und der Sensor (2) mit dem Sender in der Matratze (1) angeordnet ist.

## Beschreibung

Die Überwachung der Belegung von Räumen ist inzwischen weit verbreitet. Hintergrund für die Überwachung ist, Energieverbraucher in Abhängigkeit von der Belegung des jeweiligen Raums ein- und auszuschalten, wodurch eine erhebliche Energieersparnis erreicht werden kann. Dies hat sich insbesondere in Bürogebäuden als vorteilhaft herausgestellt, da ohne die Überwachungseinrichtungen während der üblichen Bürozeiten die Energieverbraucher, wie beispielsweise Beleuchtung, Drucker oder dergleichen dauerhaft eingeschaltet sind und daher unnötige Energie verbrauchen, soweit der jeweilige Raum nicht belegt ist.

Neben dem Bedürfnis, die Belegung von Räumen mit Personen zu überwachen, besteht ein großes Bedürfnis darin, die Benutzung von Betten überwachen zu können. Dies ist beispielsweise in Hotels ebenfalls zur Einsparung von Energie wünschenswert, andererseits ist die Überwachung insbesondere in Krankenhäusern, Alten- und Pflegeheimen von großer Bedeutung, um ein Höchstmaß an Betreuung bereitstellen zu können. Darüber hinaus tritt bei den Überwachungsvorrichtungen für Betten das Problem auf, dass durch die sich regelmäßig ändernde Lage des Benutzers des Bettes eine großflächig arbeitende Sensorik erforderlich ist.

Hier will die Erfindung Abhilfe schaffen. Der Erfindung liegt die Aufgabe zugrunde, eine Vorrichtung zur Erfassung der Belegung von Betten zu schaffen, die eine kostengünstige Überwachung der Belegung ermöglicht. Gemäß der Erfindung wird diese Aufgabe durch eine Vorrichtung zur Erfassung der Belegung von Betten, die eine Matratze aufweisen, gelöst, umfassend einen Sensor, der mit einem Sender verbunden ist und der Sensor mit dem Sender in der Matratze angeordnet ist.

Mit der Erfindung ist eine Vorrichtung zur Erfassung der Belegung von Betten geschaffen, die preiswert und zuverlässig ist. Durch die Anordnung des Sensors mit Sender in der Matratze ist eine unmittelbare Belegung des Bettes ermittelbar, was bei üblicherweise an den Bettrahmen vorgesehenen Überwachungsmöglichkeiten nicht möglich ist.

In Weiterbildung der Erfindung ist der Sensor in einem Einschub angeordnet, der in die Matratze einsetzbar ist. Die Verwendung eines Einschubs ermöglicht eine modulare Erstellung der Matratze. Es besteht so die Möglichkeit, Matratzen gleichen Typs mit oder ohne Überwachung auszuliefern. Zudem bietet die Verwendung eines Einschubs die Möglichkeit, in einfacher Weise bei einem Defekt des Senders diesen durch Austausch des gesamten Einschubs und Einsetzen eines neuen Einschubs zu wechseln.

In anderer Weiterbildung der Erfindung ist mindestens ein Druckbügel vorgesehen, der mit dem Sensor korrespondiert. Mit Hilfe des Druckbügels ist eine großflächige Überwachung der Belegung des Bettes möglich. In Abhängigkeit der Abmessungen des Druckbügels besteht die Möglichkeit, die Matratze bis zu den äußersten Enden der Matratze zu überwachen, so dass auch bei einer Positionsänderung des Benutzers die Belegung des Bettes erfasst wird. Auf die Verwendung mehrerer über die Matratze verteilter Sensoren, die miteinander gekoppelt sind, kann daher verzichtet werden, wodurch der regelungstechnische Aufwand gering ist.

Vorteilhaft sind zwei Druckbügel vorgesehen, zwischen denen der Sensor angeordnet ist. Die Verwendung von zwei Druckbügeln oberhalb und unterhalb des Sensors bietet die Möglichkeit, die Matratze wenden zu können, ohne dass die Gefahr besteht, durch das Wenden der Matratze die Überwachung negativ zu beeinflussen.

In Ausgestaltung der Erfindung besteht der Druckbügel aus Holz. Die Verwendung von Holz für den Druckbügel hat sich bewährt, da Holz ein ausreichend hohes Maß an Flexibilität und damit eine zuverlässige Auslösung des Sensors hervorruft.

In anderer Ausgestaltung der Erfindung weist der Druckbügel eine Ummantelung aus Kunststoff auf. Die Kunststoffummantelung gewährleistet, dass die aus Holz bestehenden Druckbügel keine Feuchtigkeit aufsaugen. Hierdurch ist die Lebensdauer der Druckbügel verbessert.

Bevorzugt weist der Sensor einen piezoelektrischen Wandler auf. Piezoelektrische Wandler sind in der Lage, auf sie einwirkende kinetische Energie in elektrische Energie umzuwandeln. Durch die Verwendung eines solchen Wandlers ist keine dauerhafte Stromversorgung für den Sensor und den Sender erforderlich. Dies wirkt sich insbesondere deshalb positiv aus, da die Verwendung einer dauerhaften Stromversorgung das Aussenden einer permanenten elektromagnetischen Strahlung zur Folge hätte, die das Wohlbefinden des Benutzers des Bettes negativ beeinflussen kann. Zudem sind Kranken- und Pflegebetten mit Rollen versehen und daher nicht ortsfest eingesetzt, was eine dauerhafte Stromversorgung mittels eines Netzkabelanschlusses erschweren würde.

Andere Weiterbildungen und Ausgestaltungen der Erfindung sind in den übrigen Unteransprüchen angegeben. Ein Ausführungsbeispiel der Erfindung ist in der Zeichnung dargestellt und wird nachfolgend im Einzelnen beschrieben. Es zeigen:
- Fig. 1: die schematische perspektivische Darstellung einer Matratze mit einem Einschub;
- Fig. 2: die schematische Darstellung eines Einschubs mit Sensor und Druckbügeln.

Die als Ausführungsbeispiel gewählte Vorrichtung zur Erfassung der Belegung von Betten ist in eine Matzratze 1 einsetzbar. Sie umfasst einen Sensor 2, der mit einem Sender verbunden ist. Der Sensor 2 ist mit dem Sender in der Matratze 1 angeordnet. Der Sensor 2 weist einen piezoelektrischen Wandler auf.

Im Ausführungsbeispiel ist der Sensor 2 in einem Einschub 3 angeordnet, der in die Matratze 1 einsetzbar ist. Der Einschub 3 ist vorzugsweise aus einem zu dem Material der Matratze 1 vergleichbaren Material hergestellt. Auf diese Weise führt die Verwendung der erfindungsgemäßen Vorrichtung lediglich zu einer nicht spürbaren Veränderung der Dämpfungseigenschaften der jeweiligen Matratze.

In dem Einschub 3 ist mindestens ein Druckbügel 4 vorgesehen, der mit dem Sensor 2 korrespondiert. Im Ausführungsbeispiel sind zwei Druckbügel 4 vorgesehen, von denen der eine oberhalb des Drucksensors, der andere unterhalb des Drucksensors angeordnet ist. Die Druckbügel 4 bestehen bevorzugt aus Holz und weisen eine Ummantelung aus Kunststoff auf, um zu verhindern, dass das Holz Feuchtigkeit in sich aufnimmt. In Abwandlung des Ausführungsbeispiels ist es selbstverständlich möglich, andere Materialien für die Druckbügel 4 zu verwenden. Insbesondere bieten sich hier Druckbügel aus Kunststoff oder Metall an.

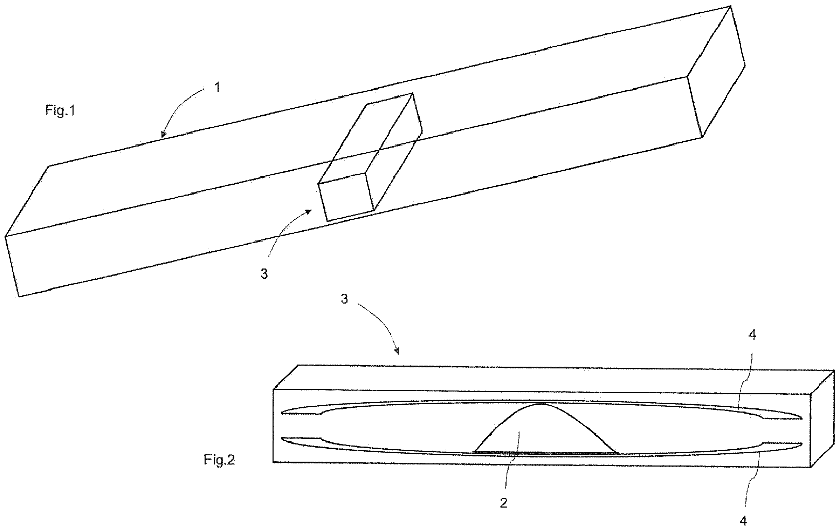

Wie Figur 2 zu entnehmen ist, ist der Sensor 2 zwischen den Druckbügeln 4 angeordnet. Die Druckbügel 4 erstrecken sich im Wesentlichen über die gesamte Breite des Einschubs 3. Da der Einschub 3 quer zur Längsmittellinie der Matratze 1 in diese eingesetzt ist, ist auf diese Weise gewährleistet, dass die gesamte Breite der Matratze 2 von dem Sensor erfasst werden kann. Dies ist dadurch ermöglicht, dass auch ein Druck auf einen der Bügel 4 am äußersten Ende des jeweiligen Bügels zu einer Auslösung des Sensors 2 führt. In Folge dessen ist gewährleistet, dass auch bei einer Positionsveränderung des jeweiligen Benutzers des Bettes eine Auslösung des Sensors erfolgt.

Der Einschub 3 hat eine im Wesentlichen quaderartige Ausbildung. Korrespondierend mit dieser Ausbildung ist in der Matratze 1 eine Öffnung vorgesehen, so dass der Einschub 3 problemlos in die Matratze 1 eingeführt werden kann. Die Ausbildung als Einschub bietet zudem den Vorteil, dass im Falle eines Defektes der Einschub ausgetauscht werden kann, um die Funktionsfähigkeit der Matratze zu gewährleisten. Es ist daher nicht erforderlich, die Matratze zu öffnen und damit den Reparaturaufwand deutlich zu erhöhen. Darüber hinaus bietet die beiderseitige und im Ausführungsbeispiel symmetrische Anordnung von zwei Druckbügeln die Möglichkeit, die Matratze zu wenden. Unabhängig davon, auf welcher Seite die Matratze auf dem jeweiligen Bettgestell aufliegt, ist eine zuverlässige Funktion der Vorrichtung sichergestellt.

Als Sensoren finden bei der erfindungsgemäßen Vorrichtung vorzugsweise piezoelektrische Wandler zum Wandeln kinetischer Energie in elektrische Energie Anwendung. Alternativ können auch elektrodynamische Energieerzeuger Anwendung finden, bei denen die Energie beispielsweise aus dem Schaltereignis selbst gewonnen wird. Auch können thermoelektrische Energieerzeuger verwendet werden, bei denen die Energie aus einer Temperaturdifferenz mittels eines Peltierelementes erzeugt wird. Als Sender finden üblicherweise Hochfrequenzsender zum drahtlosen Übertragen eines Signals Verwendung; andere Sender, beispielsweise Bluetooth- oder Infrarot-Sender können ebenfalls Verwendung finden. Sobald ein Benutzer das Bett aufsucht, führt dies zu einer Belastung des dem Benutzer zugewandten Druckbügels 4, der dadurch jedenfalls bereichsweise in Richtung des Sensors 2 bewegt wird. Dadurch wird der Sensor 2 belastet, also mit kinetischer Energie beaufschlagt, wodurch der erforderliche Strom zum Absetzen des Signals generiert wird. Der durch diese kinetische Energie geschaffene Strom ist ausreichend, um das Signal zuverlässig zu einer - nicht dargestellten - Empfangseinheit zu übermitteln. Sobald der Benutzer das Bett verlässt wird der Druckbügel 4 entlastet, was zu einer Entlastung des Sensors 2 führt. Auch hierdurch wird erneut elektrische Energie produziert, die ausreichend ist, um das erforderliche Sendesignal vom Sender in Richtung der Empfangseinheit zu übermitteln, so dass die Empfangseinheit erkennt, dass das Bett nun unbenutzt ist.

Mit dem Empfangssignal sind eine Vielzahl von Einrichtungen koppelbar. So kann beispielsweise ein Energiemanagementsystem angeschlossen sein; auch besteht die Möglichkeit, Heizung, Klimaanlage oder Licht über diese Überwachungseinrichtung zu steuern. Im Bereich der Altenpflege ist es zudem möglich, über die erfindungsgemäße Vorrichtung das Abschalten von Elektrogeräten zu ermöglichen, um zu verhindern, dass versehentlich ein Herd, ein Wasserkocher, eine Kaffeemaschine oder dergleichen über Nacht eingeschaltet bleiben. Zudem besteht im Bereich der Alten- und Krankenpflege insbesondere auch die Möglichkeit, die Belegung des Bettes durch Patienten zu überwachen und unmittelbar nach unerlaubtem Verlassen des Bettes Hilfsmaßnahmen einzuleiten.

## Patentansprüche

1. Vorrichtung zur Erfassung der Belegung von Betten, die eine Matratze (1) aufweisen, umfassend einen Sensor (2), der mit einem Sender verbunden ist, und der Sensor (2) mit dem Sender in der Matratze (1) angeordnet ist.

2. Vorrichtung nach Anspruch 1, **dadurch gekennzeichnet, dass** der Sensor (2) in einem Einschub (3) angeordnet ist, der in die Matratze (1) einsetzbar ist.

3. Vorrichtung nach einem oder mehreren der vorgenannten Ansprüche, **dadurch gekennzeichnet, dass** mindestens ein Druckbügel (4) vorgesehen ist, der mit dem Sensor (2) korrespondiert.

4. Vorrichtung nach einem oder mehreren der vorgenannten Ansprüche, **dadurch gekennzeichnet, dass** zwei Druckbügel (4) vorgesehen sind, zwischen denen der Sensor (2) angeordnet ist.

5. Vorrichtung nach einem oder mehreren der vorgenannten Ansprüche, **dadurch gekennzeichnet, dass** der Druckbügel (4) aus Holz besteht.

6. Vorrichtung nach einem oder mehreren der vorgenannten Ansprüche, **dadurch gekennzeichnet, dass** der Druckbügel (4) eine Ummantelung aus Kunststoff aufweist.

7. Vorrichtung nach einem oder mehreren der vorgenannten Ansprüche, **dadurch gekennzeichnet, dass** der Sensor (2) einen piezoelektrischen Wandler aufweist.

8. Vorrichtung nach einem oder mehreren der vorgenannten Ansprüche, **dadurch gekennzeichnet, dass** der Sensor (2) einen elektrodynamischen Energieerzeuger aufweist.

9. Vorrichtung nach einem oder mehreren der vorgenannten Ansprüche, **dadurch gekennzeichnet, dass** der Sensor (2) einen thermoelektrischen Energieerzeuger aufweist.
